# EUROPEAN PATENT APPLICATION

(11) **EP 4 575 494 A1**
(43) Date of publication of application: **25.06.2025**
(21) Application number: 24221079.7
(22) Date of filing: 18.12.2024
(51) Int. Cl.: G01N 33/487, G01N 27/74, G01N 27/447

(54) **CAPTURING A TARGET MOLECULE**

(30) Priority: 22.12.2023 EP 23219945
(71) Applicant: Imec VZW, 3001 Leuven (BE)
(72) Inventor: DALBEN MADEIRA CAMPOS, Camila, 3001 Heverlee (BE); VOS, Rita, 3128 Baal (BE); ZHANG, Lei, 3010 Kessel-Lo (BE); HOELZ, Kathrin, 3001 Leuven (BE); LIU, Chengxun, 3001 Heverlee (BE)
(74) Representative: Winger

(57) **Abstract**

In a first aspect, the present invention relates to a device (10) for capturing a target molecule, comprising: i) a first electrode (31), ii) a second electrode (32); iii) a permeable structure (50) between the first (31) and second (32) electrode, iv) a first flow channel (41) for flowing a fluid medium in between the first electrode (31) and the permeable structure (50), and v) a second flow channel (42) for flowing a fluid medium in between the second electrode (32) and the permeable structure (50); wherein the first (31) and second (32) electrode are for generating an electrophoretic force steering-in operation-the target molecule from the first flow channel (41) into the permeable structure (50), and the permeable structure (50) comprises capture sites for binding the target molecule.

## Description

### Technical field of the invention

The present invention relates to separating a target molecule (e.g. a biomolecule) in a fluid medium, and more in particular to capturing said target molecule in the fluid medium.

### Background of the invention

Separating a target molecule from a (complex) sample is a fundamental process in various scientific and clinical applications, ranging from analytical (bio)chemistry to pharmaceutical research and diagnostics. In particular, the separation of biomolecules such as nucleic acids (e.g. RNA or DNA) and proteins has seen growing interest in recent years, as a critical step towards gaining further insights in nucleic acid/protein expression, identifying potential therapeutic targets, developing new medicines, screening for diseases, etc. Such applications hinge on the ability to isolate the target molecule efficiently and rapidly.

Traditional approaches, however, are time-consuming, labour-intensive and require devices with a relatively large footprint. They therefore face limitations when dealing with high-throughput processing of large sample volumes within a constrained timeframe and physical space. In many cases, existing methods are not capable of meeting the demands of isolating substantial amounts of the target (bio)molecule from solutions with volumes in the order of tens of litres within a processing time in the order of hours. This is also apparent from the review by Huang et al. (HUANG, Shengyun, et al. Advances in capillary electrophoretically mediated microanalysis for on-line enzymatic and derivatization reactions. Electrophoresis, 2018, 39.1: 97-110.), which summarized recent developments, applications, and innovations of capillary electrophoretically mediated microanalysis methods. Their review indicates that the recent efforts have been mainly directed towards analytical applications using small sample volumes.

Satterfield et al. (SATTERFIELD, Brent C., et al. Microfluidic purification and preconcentration of mRNA by flow-through polymeric monolith. Analytical chemistry, 2007, 79.16: 6230-6235.) disclosed the trapping and concentration of eukaryotic mRNA on UV-initiated methacrylate-based porous polymer monoliths functionalized with oligodeoxythymidines (oligo-dT's). But as it uses pressure driven flow only, there is a limit on the throughput that can be reached before the pressure drop becomes prohibitive. This happens because the small pore sizes increase the pressure drop, which increase is directly proportional to the thickness of the material and consequently with the surface area that can be achieved.

There is thus still a need in the art for techniques to separate a target (bio)molecule in a (complex) sample which address at least some of the problems outlined above.

### Summary of the invention

It is an object of the present invention to provide good devices for capturing a target molecule. It is a further object of the present invention to provide good arrangements and methods associated therewith. This objective is accomplished by devices, arrangements and methods according to the present invention.

It is an advantage of embodiments of the present invention that a relatively high sample volume can processed in a high-throughput manner.

It is an advantage of embodiments of the present invention that the combination of electrophoresis with a fluid flow through the flow channels increase the sample volume which can be processed in a given timeframe. It is a further advantage of embodiments of the present invention that the fluid flow helps evacuate chemical species (e.g. gas and/or H+/OH-) formed near the electrodes. It is yet a further advantage of embodiments of the present invention that the fluid flow prevents the target molecule and/or contaminants from adsorbing to and/or reacting with the electrodes.

It is an advantage of embodiments of the present invention that the target molecule can be quickly, effectively and efficiently captured in the fluid medium, even in a complex sample.

It is an advantage of embodiments of the present invention that the capture sites can selectively bind the target molecule with respect to a further molecule. It is a further advantage of embodiments of the present invention that binding the target molecule selectively allows to separate it very efficiently from the further molecule.

It is an advantage of embodiments of the present invention that through the selection of appropriate binding sites, a large variety of molecules can be targeted, including biomolecules. It is a further advantage of embodiments of the present invention that the binding of the target molecule can be based on various physical, chemical and/or biochemical interactions.

It is an advantage of embodiments of the present invention that the target molecule can subsequently be released for further processing.

It is an advantage of embodiments of the present invention that different device architectures can be envisioned, some of which may better suited towards smaller scale while others better suited to larger (e.g. industrial scale) applications.

It is an advantage of embodiments of the present invention that multiple devices can be easily linked together to realize more involved capture/separation actions.

It is an advantage of embodiments of the present invention that the device can be easily incorporated into existing fluidic systems.

It is an advantage of embodiments of the present invention that the device/method can be readily adapted to different applications. It is a further advantage of embodiments of the present invention that it can form a key component in various processes from purification, over sensing to synthesis.

It is an advantage of embodiments of the present invention that it can be realized in a relatively straightforward and economical fashion.

It is an advantage of embodiments of the present invention that it can be realized using materials and fabrication techniques that are fairly easily accessible.

In a first aspect, the present invention relates to a device for capturing a target molecule, comprising: i) a first electrode, ii) a second electrode; iii) a permeable structure between the first and second electrode, iv) a first flow channel for flowing a fluid medium in between the first electrode and the permeable structure, and v) a second flow channel for flowing a fluid medium in between the second electrode and the permeable structure; wherein the first and second electrode are for generating an electrophoretic force steering-in operation-the target molecule from the first flow channel into the permeable structure, and the permeable structure comprises capture sites for binding the target molecule.

In a second aspect, the present invention relates to an arrangement comprising a plurality of the devices as described in any embodiment of the first aspect

In a third aspect, the present invention relates to a method for capturing a target molecule using a device or arrangement as described in any embodiment of the first or second aspect, comprising: a) providing the target molecule in a fluid medium flowing through the first flow channel; b) operating the first and second electrode to generate a force field which steers the target molecule from the first flow channel into the permeable structure; and c) capturing the target molecule by letting the target molecule bind to the capture sites.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG 1 schematically depicts an illustrative cross-section of a device in accordance with embodiments of the present invention.
FIG 2 schematically shows a 3D view of a first illustrative architecture for a device in accordance with embodiments of the present invention.
FIG 3 schematically shows a 3D view of a second illustrative architecture for a device in accordance with embodiments of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable with their antonyms under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practised without these specific details. In other instances, well-known methods, structures and techniques have not been shown in detail in order not to obscure an understanding of this description.

The following terms are provided solely to aid in the understanding of the invention.

As used herein, and unless otherwise specified, when reference is made to an electrode 'respective with' a flow channel-or vice versa-it is meant that the first electrode is respective with the first flow channel and the second electrode is respective with the second flow channel.

In a first aspect, the present invention relates to a device for capturing a target molecule, comprising: i) a first electrode, ii) a second electrode; iii) a permeable structure between the first and second electrode, iv) a first flow channel for flowing a fluid medium in between the first electrode and the permeable structure, and v) a second flow channel for flowing a fluid medium in between the second electrode and the permeable structure; wherein the first and second electrode are for generating an electrophoretic force steering-in operation-the target molecule from the first flow channel into the permeable structure, and the permeable structure comprises capture sites for binding the target molecule.

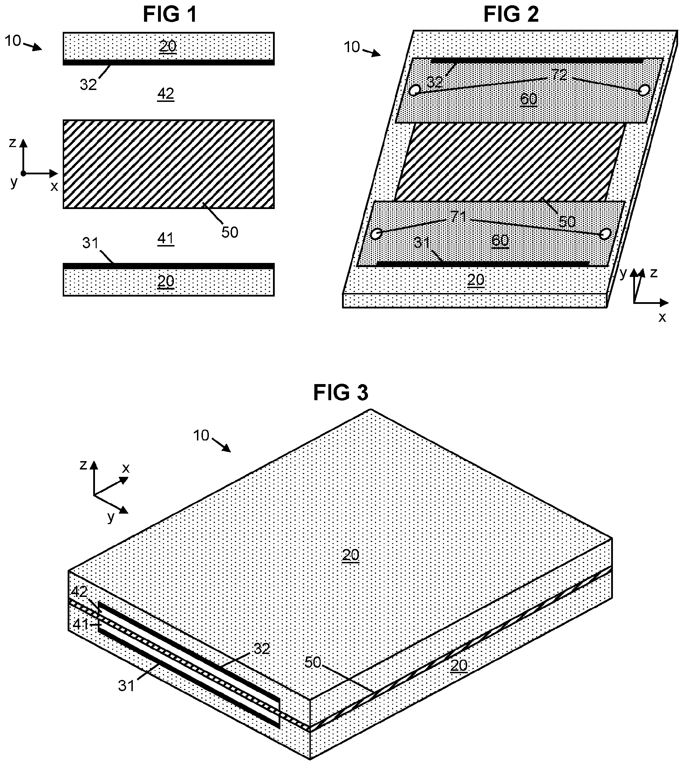

Such a device 10 may for instance have a cross-section as depicted in FIG 1, showing a first electrode 31, a first flow channel 41, a permeable structure 50, a second flow channel 42 and a second electrode 32.

In preferred embodiments, the permeable structure may comprise a metal or semiconductor nanomesh, an anodized metal or semiconductor, or a patterned material; the capture sites being comprised in said nanomesh, anodized metal or semiconductor, or patterned material.

In embodiments, the nanomesh may comprise (or consist of) a metal (e.g. Ni, Cu, Au or Pt), metal alloy or semiconductor (e.g. Si or Ge; preferably Si). The nanomesh may for instance be a nanomesh as disclosed in WO2019016033A1 (therein referred to as a "porous solid materials comprising a plurality of interconnected wires forming an ordered network"); which is incorporated herein by reference. In embodiments, the nanomesh may be coated with a dielectrice layer (e.g. silicon oxide or silicon nitride). Nanomeshes are advantageous in that the pores are interconnected (thus allowing a good flow through the material), the nanomeshes can be made free-standing (i.e. without an underlying support), can have an excellent ratio of surface area to footprint (e.g. more than 1000000) and can be made with a surrounding shell (thereby allowing to have e.g. a conductive core and an insulating surface).

In embodiments, the anodized metal or semiconductor may comprise (or consist) of an anodized metal selected from Al, Ti, Zn, Mg, Nb, Zr, Hf, Ta or Fe; preferably Al; or an anodized semiconductor selected from Si or Ge, preferably Si. Anodic aluminium oxide (AAO) is a well-known form of porous aluminium oxide, typically with a honeycomb-like structure formed by relatively dense and uniform parallel pores. Anodizing silicon likewise allows to render it porous, which pores may be similarly relatively dense, uniform and parallel, as for instance disclosed by Vyatkin et al. (VYATKIN, A., et al. Random and ordered macropore formation in p-type silicon. Journal of The Electrochemical Society, 2001, 149.1: G70.); which is incorporated herein by reference. In embodiments, the anodization may be preformed on a solid layer of the material or on a patterned material (e.g. on pillars of the material, cf. infra). Anodized metal (e.g. Al) is advantageous in that it can be made into a freestanding layer, said layer can be fairly thick and the pores can be made such that they are interconnecting; accordingly a good ratio of surface area to footprint is achievable (e.g. from about 50 to about 5000). Anodized semiconductor (e.g. Si) is advantageous in that the pore sizes can small and a good ratio of surface area to footprint is achievable (e.g. up to about 100); however, they are difficult to make free-standing (i.e. they can typically not be formed all the way through the material, but instead there typically remains a supporting layer-e.g. of the same material-underneath).

In embodiments, the patterned material may comprise (or consist) of an inorganic or organic material; preferably an inorganic material. The inorganic material may be a semiconductor (e.g. any of the semiconductors mentioned above, preferably Si), a metal (e.g. any of the metals mentioned above), an oxide (e.g. an oxide of such a semiconductor or metal, preferably SiO₂) or a nitride (e.g. a nitride of such a semiconductor or metal, preferably Si₃N₄). A patterned inorganic material may for example be obtained by lithographically etching the inorganic material through a mask (e.g. a wet or dry etching) or by a maskless lithography (e.g. electron beam, X-ray or (extreme) ultraviolet lithography). For instance, a patterned inorganic material may be obtained as disclosed in EP22216108.5; which is incorporated herein by reference. A patterned organic material may for example be obtained by one of the aforementioned lithographic techniques, or by having a block copolymer self-assemble into a periodic structure and subsequently selectively removing one type of block. The latter is also described in EP22216108.5, where this process is employed to form the mask used to etch the inorganic material. Regardless of it being an inorganic or organic material, the patterned material may comprise protrusions (i.e. material may be removed to leave in place the protrusions; e.g. pillar or fins) and/or indentations (i.e. material may be removed to form the indentations; e.g. (through) holes or trenches). In particularly preferred embodiments, the patterned material may comprise pillars (e.g. Si pillars) or through holes (e.g. etched in a Si layer). The embodiment with pillars is advantageous in that the pores therebetween are fully interconnected, and that the pillars can be manufactured relatively simply and fast. However, the achievable ratio of surface area to footprint may be modest (e.g. up to about 20), the interpillar distance relatively large (e.g. about 5 µm or more) and they are not free-standing. The embodiment with through holes is advantageous in that it can be made into a freestanding layer and that said layer can be fairly thick; they have an average ratio of surface area to footprint (e.g. to about 50). In general, Si can be a preferred material because as the backbone of the semiconductor industry, it is readily available, and its characteristics and processing are very well understood.

The first and second electrode are for generating an electric field suitable for exerting an electrophoretic force on the target molecule such that the target molecule is (re)directed (i.e. steered) from the first flow channel into the permeable structure. They are typically not particularly limited by their structure and/or make-up, other than what is needed-as is well-known in the art-to achieve the aforementioned function. For example, the electrodes typically need to be in electrical contact with (e.g. directly exposed to) the flow medium in order to generate the required electric field across the flow channels and permeable structure. The aforementioned notwithstanding, the electrodes may for example be made of Pt or another material inert to the buffer. As depicted in FIG 1, the first and second electrode 31 and 32 are provided on/against an outer surface of substrate 20 and (at least partially) define an outer edge/boundary of the first and second flow channel 41 and 42, respectively. Notwithstanding, it is in general not required that the first and second electrode are provided on/against a substrate surface. Moreover, the first and second electrodes may-independently-also protrude/extend into the respective flow channel; so that the electrode is surrounded by the flow channel (e.g. the electrode is 'in' the respective flow channel).

In embodiments, the permeable structure may comprise one or more selected from a porous structure (e.g. a membrane or mesh), protrusions (e.g. pillar or fins), beads and indentations (e.g. holes or trenches). In embodiments, the permeable structure may comprise an inorganic oxide, such as AlOₓ or SiOₓ. In alternative or complementary embodiments, the permeable structure may comprise a polymeric material (e.g. a polycarbonate membrane).

Protrusions and/or beads vs. indentations could be considered as forming a kind of continuum between two extremes; i.e. on the one hand 'islands' of the solid material (i.e. protrusions and/beads) in a 'sea' of empty space, and on the other hand 'pockets' of empty space (i.e. indentations) in a sea of solid material. In embodiments, the protrusions and/or beads may be interconnected (e.g. by means of bridges of the solid material spanning the protrusions), or the indentations may be interconnected (e.g. by means of channels in the solid material). In some embodiments, the protrusions may comprise indentations (e.g. pillars with smaller indentations), or the indentations may comprise protrusions (e.g. trenches with smaller protrusions therein).

In embodiments, the porous structure may be an (open-cell) solid foam. In embodiments, the porous structure may have a pore size of between 50 nm and 1 mm, such as between 100 nm and 300 nm. In embodiments, the porous structure may be sheets or a stack of nanostructured materials (e.g. inorganic oxides, like aforementioned) or of polymeric meshes (e.g. as currently used in filtering applications).

In some embodiments, the structure may be both porous and comprise protrusions and/or indentations. For example, the structure may comprise porous protrusions (i.e. protrusion formed of a porous material), or the indentations may be present in a porous material. In embodiments, the porous structure may comprise an anodized inorganic oxide (e.g. protrusions of or indentations in an anodized inorganic oxide).

In embodiments, the permeable structure may have a total surface area which is at least 10 times larger than a footprint of the permeable structure, preferably at least 50 times larger, more preferably at least 100 times larger, yet more preferably at least 150 times larger, most preferably at least 200 times larger; such as up to 500 times larger or even more. Here, the footprint of the permeable structure can generally be defined as the area of an orthogonal projection of the permeable structure onto a plane. This plane may for example be a surface of the substrate (cf. infra) facing the permeable structure. If no preferred plane can be selected, the footprint may be defined as the area of the largest (i.e. yielding the highest area) orthogonal projection of the structure onto a plane one can make. Thus, the footprint will generally correspond to the area defined by the outer circumference of a planar cut through the structure. This planar cut may preferably be parallel to one or more substrates (cf. infra), but may also be at an angle (i.e. different from 0°) to any or all substrates.

In embodiments, the permeable structure may be provided on a substrate and/or between substrates (e.g. between two substrates). For example, the permeable structure may be formed (e.g. by deposition, anodization, etching, etc.) on the substrate and/or between the substrates. Alternatively, the permeable structure may be a (preformed) freestanding permeable structure that is assembled on the substrate and/or between the substrates. In particular embodiments, the permeable structure may be suspended on and/or between the substrates. For example, the permeable structure may be suspended by being sandwiched at its edges between two substrates and/or by being supported on protrusions (e.g. pillars) extending from the substrate. The substrate(s) may generally be any support structure(s) on/between which the permeable structure can be stably provided. In embodiments, the substrate(s) may be (independently) made of an amorphous or (poly)crystalline material, which may be an inorganic or organic material. They could be in the form of a slide, a sheet, a plate, a wafer, a casted or moulded product, etc. The substrate(s) may for example be (independently) selected from a glass (e.g. SiOz) slide, a semiconductor (e.g. Si) wafer or a polymer sheet.

In embodiments, the first and second flow channel may each comprise an inlet and an outlet. In embodiments,-in operation-a fluid flow may flow through each flow channel. The fluid flow may typically be from the respective inlet to the respective outlet. In embodiments, the fluid flow may typically be substantially alongside (e.g. substantially parallel to) the permeable structure. The fluid flow in the first flow channel may generally be substantially independent from the fluid flow in the second flow channel. For example, even while fluid flows may be substantially parallel to one another, they may nevertheless flow in opposite directions. Likewise, the flow velocity, fluid medium, dissolved/suspended species (e.g. ions), etc. need not be the same in both flow channels.

The first and second flow channel are generally at least present between the permeable structure and the respective electrode. Notwithstanding, the first and/or electrode may simultaneously be present 'in' (e.g. protruding into/be surrounded by) the respective flow channel. Typically, the first and second flow channel are preferably in direct physical contact with the permeable structure. Moreover, the first and second flow channel are preferably at least in electrical contact with the first and second electrode (thereby enabling the electrical connection to generate the required electric field; cf. supra). For example, the first and/or second flow channel may be in direct physical contact with the respective electrode. In embodiments, the first and/or second flow channel may be at least partially defined by (e.g. in) the substate (or by/in one or more of the substrates).

Compared to using only electrophoresis, the combination of electrophoresis with a fluid flow through flow channels as defined in the present invention has several advantages, namely: i) increase the sample volume which can be processed in a given time frame; ii) evacuate chemical species (e.g. gas and/or H⁺/OH⁻) formed near the electrodes; and iii) prevent the target molecule and/or contaminants from adsorbing to and/or reacting with the electrodes.

In embodiments, any of the permeable structure, the first flow channel and the second flow channel may have a shortest dimension (e.g. a height or width) between 5 µm to 100 cm, such as at least 0.5 cm. In embodiments, any of the permeable structure, the first flow channel and the second flow channel may have a longest dimension (e.g. a length) between 5 mm to 100 cm, such as at least 5 cm.

The permeable structure comprising capture sites for binding the target molecule generally entails that the capture sites are accessible to the target molecule. Accordingly, the capture sites are typically (at least) present on a surface of the permeable structure. In embodiments, the capture sites may be for binding the target molecule by one or more selected from chemical, biochemical, covalent, electrical and magnetic binding. Various capture sites are thus possible; for example, the captures sites may be capture probes, functional groups, electrical charges and/or magnetic sites. Here, 'functional groups' is used to refer to relatively simple chemical functionalities like -OH, -COOH, - NH, -SH or aromatic groups (which may bind the target molecule covalently or by hydrogen bonding, sulphur bonding, π-interactions, Van der Waals forces, etc.); whereas 'capture probes' is used to refer to more extended (and thereby typically more specific) functionalities. The latter can for example include a member of a (bio)affinity pair (so that the affinity site corresponds to one half of the pair and the target molecule comprises the complementary part) such as: antigen-antibodies, nucleic acid pair strands (e.g. DNA/DNA, DNA/RNA, poly-dT/poly-A), enzyme-substrate, metal-coordinating affinity tag-metal ion, etc.

In preferred embodiments, the capture sites may be for binding the target molecule selectively with respect to a further molecule (e.g. a contaminant if the target molecule is an analyte or vice versa; cf. infra). This advantageously allows to separate the target molecule very effectively from the further molecule.

The target molecule may typically be any molecule which can be bound by a capture site. To name but one example, the target molecule may for instance be a pollutant in residual water. Notwithstanding, it may in particular be a biomolecule such as an oligo- or polynucleotide (e.g. DNA or RNA, especially mRNA), an oligopeptide, a protein, etc.

In some embodiments, the target molecule may be an analyte. In other embodiments, the target molecule may be a contaminant. Here, 'analyte' refers to a (bio)chemical species (e.g. a (bio)molecule) of interest, while 'contaminant' refers to a (bio)chemical species that is to be separated from the analyte. Separation of an analyte from a contaminant may thus be achieved either by capturing the analyte selectively with respect to the contaminant, or by capturing the contaminant selectively with respect to the analyte. It may often be so that an analyte is to be separated from a plurality of different contaminants; in which case it may generally be easier and/or more efficient to design a permeable structure that can capture the analyte selectively with respect to a plurality of the different contaminants, rather than a permeable structure which can capture a plurality of the different contaminants selectively with respect to the analyte. For example for this reason, it may be preferred that the analyte is the target molecule. Notwithstanding, in either case, a desired separation may also involve an arrangement comprising multiple different capture devices coupled together (e.g. in series; cf. infra).

In embodiments, the device may further comprise a condition generator for adjusting the binding conditions at the capture sites. The condition generator may for example comprise a radiant flux, temperature and/or pH controller (for adjusting the radiant flux-e.g. by UV light-, temperature and/or pH at the capture sites). Adjusting the binding conditions at the capture sites may for example be used to reverse the binding between the target molecule and the capture site (thereby releasing the target molecule), or to facilitate desorbing a further (e.g. non-specifically bound) molecule from the permeable structure; cf. infra.

In embodiments, the device may further comprise a flow generator (e.g. a pump) for flowing a fluid medium in the first and second flow channels.

In embodiments, the device may further comprise a control unit configured for instructing the first and second electrodes. Moreover, the control unit may be further configured for instructing the aforementioned flow generator and/or condition generator.

In embodiments, the device may further comprise a reservoir for collecting released unbound molecules (e.g. analyte if the target molecule is a contaminant, or contaminant if the target molecule is an analyte) and/or target molecules. In embodiments, the reservoir may be fluidically coupled to the outlet of the second flow channel.

In embodiments, any feature of any embodiment of the first aspect may independently be as correspondingly described for any embodiment of any of the other aspects.

In a second aspect, the present invention relates to an arrangement comprising a plurality of the devices as described in any embodiment of the first aspect.

In embodiments, the plurality of the devices may be fluidically coupled in parallel and/or in series.

Note that in such an arrangement, it is not necessary (and indeed: it may often not be the case) that the target molecule is the same in each device. In particular, the permeable structures of different devices may be adapted for capturing different target molecules. For example, an arrangement could be set up so that an analyte passes through a plurality of the capture devices in each of which one or more contaminants are captured (and thus separated from analyte). Likewise, an arrangement could be set up so that an analyte passes through a plurality of the capture devices in which it is the target molecule in some (but not in others) or in all of the capture devices. Moreover, an arrangement could be set up having multiple paths/branches, each of which may be independently configured for different analytes.

In embodiments, the arrangement may further comprise a flow generator (e.g. a pump) for flowing a fluid medium in the first and second flow channels.

In embodiments, the arrangement may further comprise a control unit configured for instructing the first and second electrodes. Moreover, the control unit may be further configured for instructing the aforementioned flow generator.

In embodiments, any feature of any embodiment of the second aspect may independently be as correspondingly described for any embodiment of any of the other aspects.

In a third aspect, the present invention relates to a method for capturing a target molecule using a device or arrangement as described in any embodiment of the first or second aspect, comprising: a) providing the target molecule in a fluid medium flowing through the first flow channel; b) operating the first and second electrode to generate a force field which steers the target molecule from the first flow channel into the permeable structure; and c) capturing the target molecule by letting the target molecule bind to the capture sites.

In preferred embodiments, step c may performed selectively with respect to a further molecule. Accordingly, step c may comprise letting the further molecule pass through (or adsorb non-specifically to) the permeable structure.

In embodiments, the method may comprise a further step d-after step c-of: d) desorbing (e.g. by adjusting temperature and/or pH) a further (e.g. non-specifically bound) molecule from the permeable structure.

In embodiments, the method may comprise a further step e-after step d, if present-of: e) releasing the target molecule by reversing (e.g. by adjusting temperature or using UV light) the binding between the target molecule and the capture sites.

In embodiments, the method may further comprise collecting released unbound molecules and/or target molecules (cf. supra).

In embodiments, the method may comprise further processing the target molecule and/or the captured molecule (e.g. further processing the analyte). While the present invention generally deals with capturing a target molecule (and thereby typically separating it from the fluid and/or further species in the fluid), this is not necessarily the end goal and the method can be readily extended towards various processes involving purification, sensing, synthesis, etc.

In embodiments, any feature of any embodiment of the third aspect may independently be as correspondingly described for any embodiment of any of the other aspects.

The invention will now be described by a detailed description of several embodiments of the invention. It is clear that other embodiments of the invention can be configured according to the knowledge of the person skilled in the art without departing from the true technical teaching of the invention, the invention being limited only by the terms of the appended claims.

### Example 1

We now refer to FIG 2, showing a first illustrative example of a device 10 in accordance with the present invention. The cross-section of this device 10 may be as depicted in FIG 1.

The instant device 10 comprises a substrate 20 on which a permeable structure 50 is provided. The permeable structure 50 may for example be an arrangement of pillars or pillar-like structure, e.g. as obtainable by anodization. In particular, the pillars may stand on (`be sustained by') the substrate and be substantially aligned in the y-direction; although other arrangements and/or alignments are also possible. The permeable structure 50 (e.g. the pillars) may preferably span at least the channel height (cf. infra). The permeable structure 50 comprises capture sites at its surface (for capturing the target molecule), which may be capture probes, functional groups, electrical charges and/or magnetic sites.

Also present are a pair of electrodes 31 and 32 on opposites sides of the permeable structure 50 and two flow channels (not directly visible in FIG 2), respectively between the first electrode 31/second electrode 32 and the permeable structure 50. Covers 60 are further provided to seal the flow channels, thereby defining the channel height (along the y-direction) between the substrate and the cover; the substrate and cover respectively forming a channel bottom and channel ceiling. Optionally, also the permeable structure 50 may be sealed by a cover (e.g. a single cover over the permeable structure 50 and the flow channels).

Also depicted in FIG 2 are a pair of in- and outlets for each flow channel (pair 71 for the first flow channel and pair 72 for the second flow channel).

The above device architecture may generally be better suited towards smaller-scale devices, e.g. for use in a lab or as point-of-care devices. Typical dimensions for the permeable structure and each flow channel may for example be a height (along the y-direction) from about 20 µm to the order of millimetres, a width (along the z-direction) of a few micrometres to in the order of centimetres, and a length (along the x-direction) of in the order of millimetres to in the order of centimetres.

In operation, a fluid flow is generated in each flow channel (from the respective inlet to the respective outlet; i.e. substantially alongside/parallel to the permeable structure 50). Simultaneously, the first electrode 31 and second electrode 32 are operated to generate a suitable electrophoretic field across the permeable structure 50. Upon providing the target molecule in the fluid medium in the first flow channel, an electrophoretic force thereby acts on the target molecule, steering it from the first flow channel into the permeable structure 50. Inside the permeable structure 50, the target molecule then (preferably selectively) interacts with-and is captured by-the captures sites, thereby separating it from the rest of the fluid species. More specifically, the fluid and other species (e.g. contaminants) which are not (sufficiently) affected by the electrophoretic field continue to flow through the first flow channel and can be collected at the first outlet. On the other hand, the species (e.g. contaminants) which are steered into the permeable structure, but which do not bind with the capture sites flow through the permeable structure and can-through the second flow channel-be collected at the second outlet.

Depending on the specifics of the case (e.g. different materials involved, desired separation, etc.), it may be possible that a non-trivial amount of further species (i.e. otherthan the target molecule) becomes (reversibly) adsorbed to the permeable structure 50 (to the captures sites as such or otherwise). However, the binding affinity of these species is generally lower than that of the target molecule, so that it is possible to desorb them while keeping the target molecule bound. To promote this, it can be useful to (slightly) vary the conditions (e.g. temperature and/or pH) in the device.

Finally, where the capture sites are selected such that the binding with the target molecule is reversible, the target molecule can be released when desired (depending on the capture site, based on temperature or using UV light) and collected at the second outlet.

### Example 2

We now refer to FIG 3, showing a second illustrative example of a device 10 in accordance with the present invention. The cross-section of this device 10 may also be as depicted in FIG 1.

The instant device 10 comprises a freestanding permeable structure 50-like a membrane-which is assembled between two substrates 20. The permeable structure 50 is thereby suspended by being sandwiched at its edges between two substrates 20. Optionally, small pillars may be provided (e.g. one or both of the substrates 20 may be outfitted therewith) under and/or above the permeable structure 50 to further support/sustain it. The freestanding permeable structure (e.g. membrane) 50 may preferably be made of a porous material, e.g. with a pore size of at least 50 nm. For example, the porous material may be sheets or a stack of nanostructured materials (e.g. inorganic oxides, such as AlOₓ or SiOₓ) or of polymeric meshes (e.g. as currently used in filtering applications). A treatment (e.g. a plasma or UV treatment) may be applied to the porous material to generate (additional) functional groups on the surface of the porous material. Depending on the application, these functional groups can be used directly as capture site or can be used to bind a capture probe thereto.

Each of the two substrates 20 are further provided with a cavity and an electrode 31 or 32. Accordingly,-after assembly-the permeable structure 50 is between the pair of electrodes 31 and 32, and two flow channels 41 and 42 are defined (respectively between the first electrode 31/second electrode 32 and the permeable structure 50).

The above device architecture may generally be better suited towards larger-scale devices, e.g. for industrial applications. Typical dimensions for the permeable structure and each flow channel may for example be a height (along the z-direction) of in order of tens of micrometres to about 1 cm, and a width (i.e. along the y-direction) and length (i.e. along the x-direction) of in the order of micrometres to tens of centimetres.

Like the device of Example 1,-in operation-a fluid flow is generated in each flow channel 41 and 42, a suitable electrophoretic field is generated across the permeable membrane with electrodes 31 and 32, and the operation for separating the target molecule is substantially similar as in Example 1.

It is to be understood that although preferred embodiments, specific constructions, configurations and materials have been discussed herein in order to illustrate the present invention. It will be apparent to those skilled in the art that various changes or modifications in form and detail may be made without departing from the scope of the invention as defined in the appended claims.

## Claims

1. A device (10) for capturing a target molecule, comprising:
i) a first electrode (31),
ii) a second electrode (32);
iii) a permeable structure (50) between the first (31) and second (32) electrode,
iv) a first flow channel (41) for flowing a fluid medium in between the first electrode (31) and the permeable structure (50), and
v) a second flow channel (42) for flowing a fluid medium in between the second electrode (32) and the permeable structure (50);
wherein
- the first (31) and second (32) electrode are for generating an electrophoretic force steering-in operation-the target molecule from the first flow channel (41) into the permeable structure (50), and
- the permeable structure (50) comprises capture sites for binding the target molecule.

2. The device (10) according to claim 1, wherein the permeable structure (50) has a total surface area which is larger than a footprint of the permeable structure (50), preferably at least 5 times larger, more preferably 10 times larger, yet more preferably at 20 times larger, most preferably at least 50 times larger.

3. The device (10) according to claim 1 or 2, wherein the permeable structure (50) comprises one or more selected from a porous structure, protrusions and beads.

4. The device (10) according to any of the previous claims, wherein the permeable structure (50) comprises an inorganic oxide.

5. The device (10) according to any of the previous claims, wherein any of the permeable structure (50), the first flow channel (41) and the second flow channel (42) has:
- a shortest dimension between 5 µm to 100 cm, and/or
- a longest dimension between 5 mm to 100 cm.

6. The device (10) according to any of the previous claims, wherein the capture sites are for binding the target molecule by one or more selected from chemical, biochemical, covalent, electrical and magnetic binding.

7. The device (10) according to any of the previous claims, wherein the capture sites are for binding the target molecule selectively with respect to a further molecule.

8. The device (10) according to any of the previous claims, further comprising a condition generator for adjusting the binding conditions at the capture sites.

9. The device (10) according to any of the previous claims, further comprising a reservoir for collecting released target molecules or unbound molecules.

10. The device (10) according to any of the previous claims further comprising:
- a flow generator for flowing a fluid medium in the first and second flow channels (42), and
- a control unit configured for instructing the flow generator and the first (31) and second (32) electrodes.

11. An arrangement comprising a plurality of the devices (10) according to any of the previous claims.

12. The arrangement according to claim 11 as depending on any of the claims 1 to 9, further comprising:
- a flow generator for flowing a fluid medium in the first and second flow channels (42) of the plurality of devices (10), and
- a control unit configured for instructing the flow generator and the first (31) and second (32) electrodes of the plurality of devices (10).

13. A method for capturing a target molecule using a device (10) or arrangement as defined in any of the previous claims, comprising:
a) providing the target molecule in a fluid medium flowing through the first flow channel (41);
b) operating the first (31) and second (32) electrode to generate a force field which steers the target molecule from the first flow channel (41) into the permeable structure (50); and
c) capturing the target molecule by letting the target molecule bind to the capture sites.

14. The method according to claim 13, comprising a further step d-after step c-of:
d) desorbing a further molecule from the permeable structure (50).

15. The method according to any of claims 13 to 14, comprising a further step e-after step d, if present-of:
e) releasing the target molecule by reversing the binding between the target molecule and the capture sites.
